# EUROPEAN PATENT APPLICATION

(11) **EP 2 842 495 A1**
(43) Date of publication of application: **04.03.2015**
(21) Application number: 14165678.5
(22) Date of filing: 23.04.2014
(51) Int. Cl.: A61B 8/00, A61B 5/06, A61B 8/08

(54) **Method of generating body marker and ultrasound diagnosis apparatus using the same**

(30) Priority: 26.08.2013 KR 20130101282
(71) Applicant: Samsung Medison Co., Ltd., Gangwon-do 250-870 (KR)
(72) Inventor: Yoon, Ki-sang, Hongcheon-gun 250-870 Gangwon-do (KR); Jo, Jae-moon, Hongcheon-gun 250-870 Gangwon-do (KR); Oh, Dong-hoon, Hongcheon-gun 250-870 Gangwon-do (KR); Hyun, Dong-gyu, Hongcheon-gun 250-870 Gangwon-do (KR)
(74) Representative: Schmid, Wolfgang

(57) **Abstract**

A body marker generation method and an ultrasound diagnosis apparatus using the same are provided. The body marker generation method includes capturing an image of a subject by using a three-dimensional (3D) infrared sensor; acquiring position information indicating a position of a probe with respect to the subject; determining a diagnostic part of the subject based on the position information, the diagnostic part being a target object to which the probe transmits an ultrasonic signal; and displaying a body marker for the diagnostic part.

## Description

### CROSS-REFERENCE TO RELATED PATENT APPLICATION

This application claims the benefit of Korean Patent Application No. 10-2013-0101282, filed on August 26, 2013 in the Korean Intellectual Property Office, the disclosure of which is incorporated herein in its entirety by reference.

### BACKGROUND OF THE INVENTION

### 1. Field of the Invention

One or more embodiments of the present invention relate to a method of generating a body marker and an ultrasound diagnosis apparatus using the same.

### 2. Description of the Related Art

An ultrasound diagnosis apparatus uses a probe to generate an ultrasound signal (generally, greater than 20 kHz) directed toward a predetermined part inside an object, and obtains an image of the predetermined part by using a reflected echo signal. Ultrasound diagnosis apparatuses are used for medical purposes, such as detection of impurities in a body, measurement and observation of wounds, etc. Compared to X-ray apparatuses, the ultrasound diagnosis apparatuses have various advantages such as real-time display of images and increased safety due to no radioactive exposure. Thus, the ultrasound diagnosis apparatuses are commonly used together with other image diagnosis apparatuses.

The image (hereinafter referred to as the ultrasound image) may be displayed on a display of the ultrasound diagnosis apparatus or may be stored in a storage medium and displayed on other apparatuses. For example, the size of the ultrasound image may be reduced and the reduced ultrasound image may be displayed on a screen of a cellular phone, portable electronic device, personal digital assistant (PDA), tablet PC, etc.

### SUMMARY OF THE INVENTION

One or more embodiments of the present invention include a method of generating a body marker indicating an anatomical position of a diagnostic part during ultrasound diagnosis.

One or more embodiments of the present invention also include an apparatus for generating a body marker indicating an anatomical position of a diagnostic part during ultrasound diagnosis.

One or more embodiments of the present invention also include a computer readable recording medium having embodied thereon a computer program for executing the method.

According to one or more embodiments of the present invention, a body marker generation method used by an ultrasound diagnosis apparatus includes: capturing an image of a subject by using a three dimensional (3D) infrared sensor; acquiring position information indicating a position of a probe with respect to the subject; determining a diagnostic part of the subject based on the position information, the diagnostic part being a target object to which the probe transmits an ultrasonic signal; and displaying a body marker for the diagnostic part.

The acquiring may include measuring at least one of a linear movement direction of the probe, a scan direction of the probe, and a rotation direction of the probe.

The acquiring may include: receiving a plurality of infrared signals through the 3D infrared sensor, the infrared signals being emitted from a plurality of infrared light emitting diodes (LEDs) attached on the probe; and measuring the position information from the plurality of infrared signals.

The acquiring may include: transmitting an infrared signal by using the 3D infrared sensor; receiving a reflection signal generated when the infrared signal is reflected by an infrared reflective sheet attached on the probe; and measuring the position information from the reflection signal.

The infrared reflective sheet may include a pattern for reflecting the infrared signal to generate the reflection signal.

The capturing may include: sensing a plurality of feature points of the subject; and connecting the plurality of feature points to each other to acquire a frame structure of the subject.

The body marker generation method may further include dividing the frame structure into a plurality of segments, wherein the acquiring includes matching the position of the probe to any one of the plurality of segments.

The acquiring may include acquiring the position information through a 3D labeling process for the probe.

The acquiring may include comparing a first image captured from the subject in advance and a second image captured from both the probe and the subject to acquire the position information.

The body marker generation method may further include acquiring probe information indicating at least one of an arrangement of a transducer included in the probe, the type of the probe, and the purpose of the probe, wherein the determining includes determining the diagnostic part based on the position information and the probe information.

The displaying may include displaying an image indicating the diagnostic part as the body marker.

The displaying may include displaying an image indicating the diagnostic part and an image indicating the probe as the body marker.

The displaying may include displaying an image in which the diagnostic part is distinguished visually from other parts in an exemplary image of the subject, as the body marker.

The 3D infrared sensor may be attached to a diagnostic table or a diagnostic chair on which the subject is positioned.

According to one or more embodiments of the present invention, an ultrasound diagnosis apparatus for generating a body marker includes; an infrared imaging unit for capturing an image of a subject by using a three dimensional (3D) infrared sensor; an image analyzer for acquiring position information indicating a position of a probe with respect to the subject and for determining a diagnostic part of the subject based on the position information, the diagnostic part being a target object to which the probe transmits an ultrasonic signal; an image processor for generating a body marker for the diagnostic part; and a display unit for displaying the body marker.

According to one or more embodiments of the present invention, a non-transitory computer-readable recording medium having recorded thereon a program for executing the method is provided.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a system related to an ultrasound diagnosis apparatus, according to an embodiment of the present invention;
FIG. 2 is a block diagram illustrating an ultrasound diagnosis apparatus according to an embodiment of the present invention.
FIG. 3 is a block diagram illustrating an ultrasound diagnosis apparatus according to another embodiment of the present invention;
FIG. 4 is a flowchart illustrating a method of generating a body marker, according to an embodiment of the present invention;
FIG. 5 is a flowchart illustrating a method of generating a body marker, according to another embodiment of the present invention;
FIGS. 6A and 6B are diagrams illustrating processes of acquiring position information about the position of a probe, according to embodiments of the present invention;
FIG. 7 is a diagram illustrating a process of acquiring position information about the position of a probe, according to another embodiment of the present invention;
FIGS. 8A and 8B are diagrams illustrating processes of acquiring position information about the position of a probe, according to other embodiments of the present invention;
FIGS. 9A and 9B are diagrams illustrating processes of determining a diagnostic part, according to an embodiment of the present invention;
FIGS. 10A through 10C are diagrams illustrating processes of displaying a body marker, according to an embodiment of the present invention; and
FIG. 11 is a diagram illustrating an arrangement of a 3D infrared sensor, according to an embodiment of the present invention.

### DETAILED DESCRIPTION OF THE INVENTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

Terms used in the present invention have been selected as general terms which are widely used at present, in consideration of the functions of the present invention, but may be altered according to the intent of an operator skilled in the art, conventional practice, or introduction of new technology. Also, if there is a term which is arbitrarily selected by the applicant in a specific case, the meaning of the term will be described in detail in a corresponding description portion of the present invention. Therefore, the terms should be defined on the basis of the entire content of this specification instead of a simple name of each of the terms.

In this disclosure, when it is described that one part comprises (or includes or has) some elements, it should be understood that the part may comprise (or include or has) only those elements, or it may comprise (or include or have) other elements as well as those elements if there is no specific limitation. The term "module", as used herein, means, but is not limited to, a software or hardware component, such as a Field Programmable Gate Array (FPGA) or an Application Specific Integrated Circuit (ASIC), which performs certain tasks. A module may advantageously be configured to reside in an addressable storage medium and configured to execute on one or more processors.

Thus, a module may include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. The functionality provided for in the components and modules may be combined into fewer components and modules or further separated into additional components and modules.

The term "image" used herein may denote multi-dimensional data composed of discrete image factors (for example, pixels in a two-dimensional (2D) image and pixels in a three-dimensional (3D) image). For example, an image may include a medical image of an object which is acquired by an X-ray apparatus, a computed tomography (CT) apparatus, a magnetic resonance imaging (MRI) apparatus, an ultrasound apparatus, or another medical diagnosis system.

Moreover, the term "subject" or "object" used herein may be a person, an animal, a part of a person, or a part of an animal. For example, an object may be an organ such as a liver, a heart, a womb, a brain, breasts, an abdomen, or the like, or a blood vessel. Also, the term "object" may be a phantom. The phantom denotes a body having a density very close to a density of human organs and a similar effective atomic number, and may be a spherical phantom having a shape similar to a human body.

Moreover, the term "user" used herein indicates a medical expert such as a medic, a nurse, a medical technologist, a medical image expert, or the like, or an engineer that maintains a medical apparatus. However, the user is not limited thereto.

Hereinafter, embodiments of the present invention will be described in detail to be easily embodied by those of ordinary skill in the art with reference to the accompanying drawings. The present invention may, however, be embodied in many different forms and should not be construed as being limited to the embodiments set forth herein. In the accompanying drawings, a portion irrelevant to a description of the present invention will be omitted for clarity. Moreover, like reference numerals refer to like elements throughout.

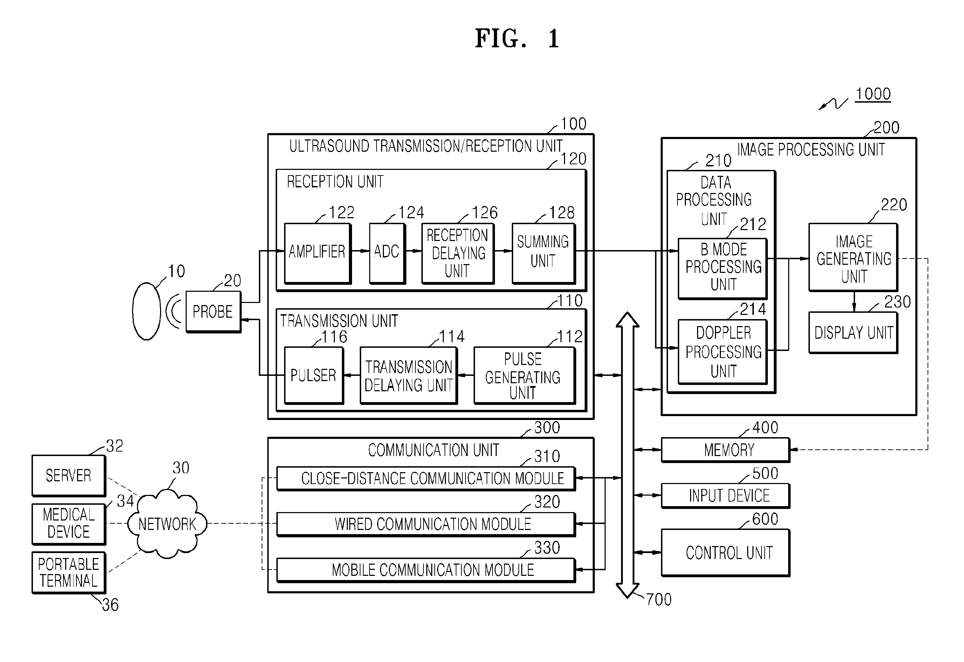

FIG. 1 is a block diagram illustrating a system related to an ultrasound diagnosis apparatus 1000, according to an embodiment of the present invention.

Referring to FIG. 1, the ultrasound diagnosis apparatus 1000 may include a probe 20, an ultrasonic transceiver 100, an image processor 200, a communicator 300, a memory 400, an input device 500, and a controller 600. The above-described elements may be connected to each other through a bus 700.

The ultrasound diagnosis apparatus 1000 may be a portable type apparatus as well as a cart type apparatus. Examples of the portable ultrasound diagnosis apparatuses may include picture archiving and communication system (PACS) viewers, smartphones, laptop computers, personal digital assistants (PDAs), tablet personal computers (PCs), etc., but are not limited thereto.

The probe 20 sends an ultrasonic signal to a target object 10 according to a driving signal applied from the ultrasonic transceiver 100, and receives an echo signal reflected from the target object 10. The probe 20 includes a plurality of transducers which vibrate according to the applied driving signal to generate an ultrasonic wave, that is, sound energy. Also, the probe 20 may be connected to a body of the ultrasound diagnosis apparatus 1000 in a wired or wireless manner, and the ultrasound diagnosis apparatus 1000 may include a plurality of the probes 20 depending on an implementation type.

A transmission unit 110 supplies the driving signal to the probe 20, and includes a pulse generator 112, a transmission delayer 114, and a pulser 116. The pulse generator 112 generates a pulse that is used for forming a transmission ultrasonic wave based on a pulse repetition frequency (PRF), and the transmission delayer 114 applies a delay time to the pulse in order to determine a transmission directionality thereof. A plurality of the pulses with the delay time applied thereto respectively correspond to a plurality of piezoelectric vibrators included in the probe 20. The pulser 116 applies the driving signal (or a driving pulse) to the probe 20 at a timing corresponding to each of the pulses with the delay time applied thereto.

The reception unit 120 processes the echo signal received from the probe 20 to generate ultrasonic data, and may include an amplifier 122, an analog-to-digital converter (ADC) 124, a reception delayer 126, and an adder 128. The amplifier 122 amplifies the echo signal for each channel, and the ADC 124 converts the amplified echo signal from analog to digital. The reception delayer 126 applies a delay time to the digital-converted echo signal in order to determine a reception directionality thereof, and the adder 128 adds a plurality of the echo signals processed by the reception delayer 166 to generate the ultrasonic data. The reception unit 120 may not include the amplifier 122 depending on an implementation type. That is, the amplifier 122 may be omitted when the sensitivity of the probe 20 is improved or the number of processing bits of the ADC 124 is increased.

The image processor 200 performs a scan conversion on the ultrasonic data generated by the ultrasonic transceiver 100 to generate and display an ultrasonic image. The ultrasonic image may include a Doppler image representing a moving object by using a Doppler effect as well as a grayscale ultrasonic image acquired by scanning the target object in an amplitude (A) mode, a brightness (B) mode, and a motion (M) mode. The Doppler image may include a blood Doppler image (also called a color Doppler image) indicating a flow of blood, a tissue Doppler image indicating a motion of a tissue, and a spectral Doppler image that displays a moving speed of the target object as a waveform.

A B mode processor 212 extracts a B mode component from the ultrasonic data to process the B mode component. An image generation unit 220 may generate, on the basis of the B mode component extracted by the B mode processor 212, an ultrasonic image that displays signal intensity as brightness.

Similarly, a Doppler processor 214 may extract a Doppler component from the ultrasonic data, and the image generation unit 220 may generate a Doppler image that displays a motion of a target object as a color or a waveform, on the basis of the extracted Doppler component.

The image generation unit 220 according to an embodiment may perform a volume rendering operation on volume data to generate a 3D ultrasonic image, and may also generate an elastic image that displays a degree of modification (based on a pressure) of a target object 10 as an image. Furthermore, the image generation unit 220 may express various pieces of additional information on the ultrasonic image as texts and graphics. The generated ultrasonic image may be stored in a memory 400.

A display unit 230 displays the generated ultrasonic image. In addition to the ultrasonic image, the display unit 230 may display various pieces of information processed by the ultrasound diagnosis apparatus 1000 on a screen through a graphics user interface (GUI). The ultrasound diagnosis apparatus 1000 may include two or more display units 230 depending on an implementation type.

The communicator 300 is connected to a network 30 in a wired or wireless manner to communicate with an external device or server. The communicator 300 may exchange data with a hospital server or a medical apparatus of a hospital which is connected thereto through a medical image information system (PACS). Also, the communicator 300 may perform data communication according to the digital imaging and communications in medicine (DICOM) standard.

The communicator 300 may transmit and receive data, such as an ultrasonic image, ultrasonic data, Doppler data, etc. of the target object 10, associated with a diagnosis of the target object over the network 30, and may also transmit and receive a medical image captured by a medical apparatus such as a computed tomography (CT) apparatus, a magnetic resonance imaging (MRI) apparatus, or an X-ray apparatus. Furthermore, the communicator 300 may receive information on a diagnosis history or treatment schedule of a patient from a server, and use a diagnosis of the target object 10. In addition, the communicator 300 may perform data communication with a portable terminal of a doctor or a patient, in addition to a server or medical apparatus of a hospital.

The communicator 300 may be connected to the network 30 in a wired or wireless manner, and may exchange data with a server 32, a medical apparatus 34, or a portable terminal 36. The communicator 300 may include one or more elements that enable communication with an external device. For example, the communicator 300 includes a short-distance communication module 310, a wired communication module 320, and a mobile communication module 330.

The short-distance communication module 310 is a module for short-distance communication. According to an embodiment of the present invention, the short-distance communication may be performed via wireless LAN, Wi-Fi, Bluetooth, Zigbee, Wi-Fi direct (WFD), ultra wideband (UWB), or infrared data association (IrDA), Bluetooth low energy (BLE), and may also be near field communication (NFC). However, the present invention is not limited to the above examples of short-distance communication.

The wired communication module 320 is a module for communication using an electrical signal or an optical signal. Wired communication according to an embodiment may be performed via a pair cable, a coaxial cable, an optical fiber cable, an Ethernet cable, etc.

The mobile communication module 330 transmits and receives a radio frequency (RF) signal to and from a base station, an external terminal, and a server over a mobile communication network. The RF signal may include various types of data based on transmission and reception of a voice call signal, a video call signal, or a letter/multimedia message.

The memory 400 stores various pieces of information processed by the ultrasound diagnosis apparatus 1000. For example, the memory 400 may store medical data, such as input/output ultrasonic data and ultrasonic images, associated with a diagnosis of a target object, and may also store an algorithm or a program which is executed in the ultrasound diagnosis apparatus 1000.

The memory 400 may be a flash memory, a hard disk, an EEPROM, etc. Also, the ultrasound diagnosis apparatus 1000 may operate a web storage or a cloud server which performs a storage function of the memory 400 on a web.

The input device 500 receives data for controlling the ultrasound diagnosis apparatus 1000 from a user. The input device 500 may include hardware elements such as a keypad, a mouse, a touch panel, a touch screen, a trackball, a jog switch, but is not limited thereto. As another example, the input device 500 may further include various input units such as an electrocardiogram (ECG) measurement module, a breath measurement module, a voice recognition sensor, a gesture recognition sensor, a fingerprint recognition sensor, an iris recognition sensor, a depth sensor, a distance sensor, etc.

The controller 600 controls an overall operation of the ultrasound diagnosis apparatus 1000. That is, the controller 600 may control operations between the probe 20, the ultrasonic transceiver 100, the image processor 200, the communicator 300, the memory 400, and the input device 500 which are illustrated in FIG. 1.

Some or all of the probe 20, the ultrasonic transceiver 100, the image processor 200, the communicator 300, the memory 400, the input device 500, and the controller 600 may be operated by a software module, but the present invention is not limited thereto. Some of the above-described elements may be operated by a hardware module. Also, at least some of the ultrasonic transceiver 100, the image processor 200, and the communicator 300 may be included in the controller 600, but are not limited to the implementation type.

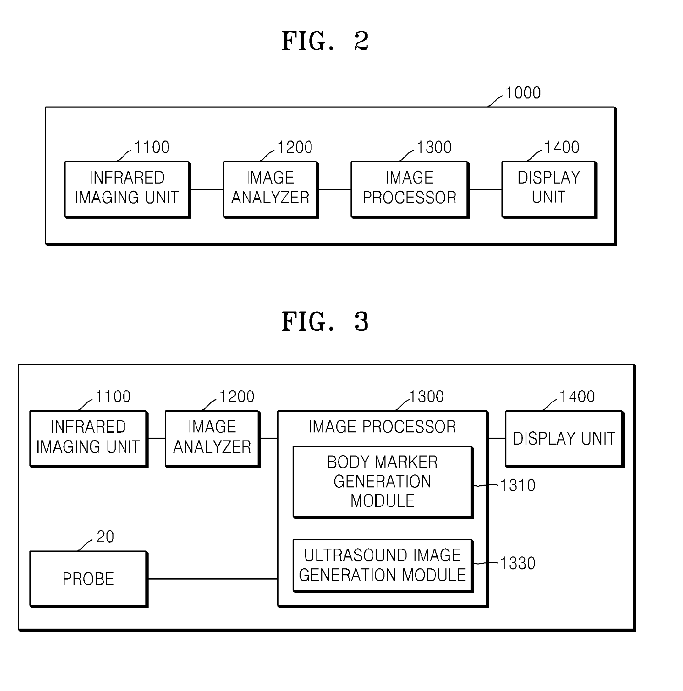

FIG. 2 is a block diagram illustrating an ultrasound diagnosis apparatus 1000 according to an embodiment of the present invention.

Referring to FIG. 2, the ultrasound diagnosis apparatus 1000 may include an infrared imaging unit 1100, an image analyzer 1200, an image processor 1300, and a display unit 1400. The ultrasound diagnosis apparatus 1000 may further include other elements in addition to the elements illustrated in FIG. 2.

The infrared imaging unit 1100 uses an infrared sensor to capture an image of a subject. The infrared imaging unit 1100 may include a 3D infrared sensor that transmits and receives an infrared signal. That is, the infrared imaging unit 1100 may use the 3D infrared sensor to transmit the infrared signal to the subject, and may receive a reflective infrared signal to capture the image of the subject.

The 3D infrared sensor may recognize a target object reflecting an infrared signal as a section thereof, like a 2D infrared sensor, and may also recognize the target object reflecting the infrared signal in three dimensions. That is, the 3D infrared sensor may acquire information about a depth defined as a distance from the 3D infrared sensor to the target object.

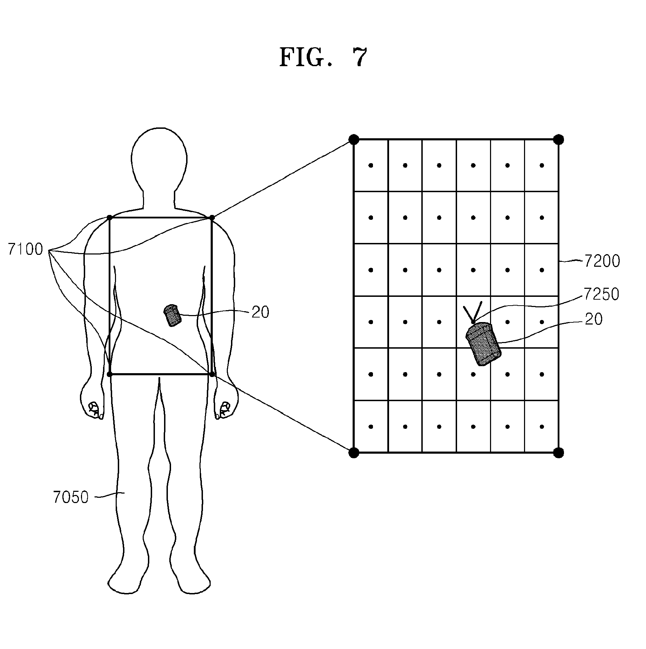

In an embodiment, the infrared imaging unit 1100 may use the infrared sensor to sense a feature point of the subject. The feature point may mean one or more points used by the ultrasound diagnosis apparatus 1000 to recognize an anatomical body structure of the subject. For example, the infrared imaging unit 1100 may sense two positions of both shoulders of the subject and two positions of the pelvis of the subject as the feature points. An embodiment related to the feature points will be described in detail with reference to FIG. 7.

In another embodiment, the infrared imaging unit 1100 may recognize a probe by using an infrared signal. For example, the infrared imaging unit 1100 may recognize the probe from one or more infrared light emitting diode (LED) attached to the probe. That is, the infrared imaging unit 1100 may receive an infrared signal emitted from the one or more infrared LED, and the image analyzer 1200 may recognize the probe based on an angle at which the infrared signal is received and the intensity of the infrared signal.

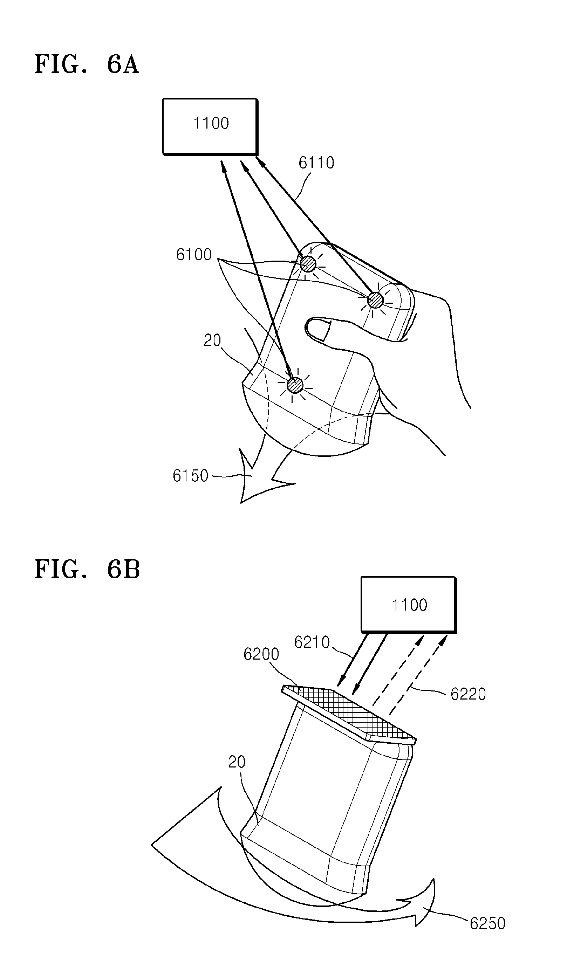

In another embodiment, the infrared imaging unit 1100 may emit an infrared signal, and may receive a signal reflected from an infrared reflective sheet attached to a probe. That is, the infrared imaging unit 1100 may transmit the infrared signal to recognize a position of the probe and may receive the reflected signal. The image analyzer 1200 may analyze the reflected signal to recognize the probe, and this operation will be described with reference to FIG. 6.

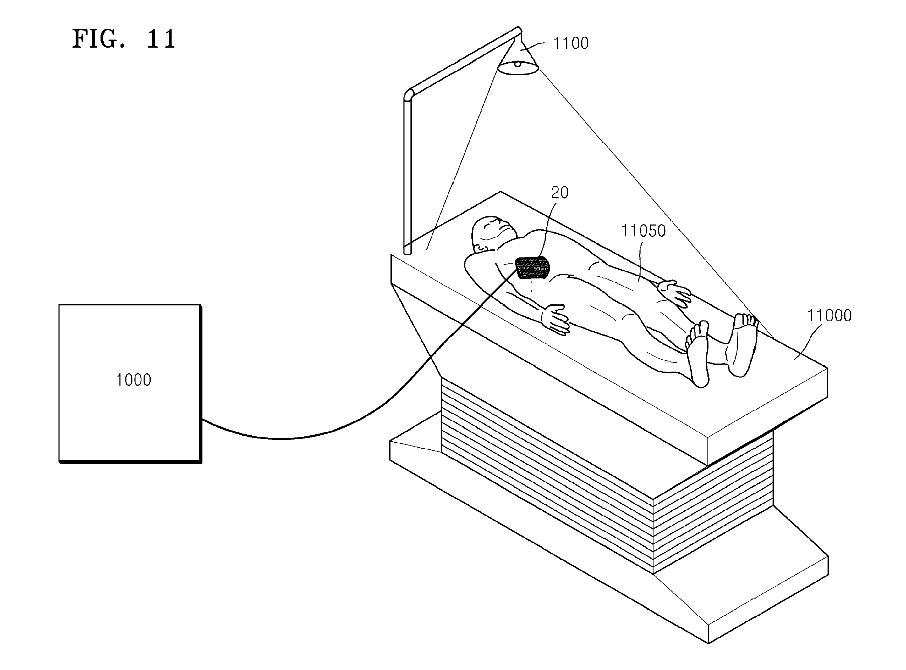

The infrared imaging unit 1100 may be attached to a diagnostic table or a diagnostic chair on which a subject is positioned. That is, the infrared imaging unit 1100 may be connected to the diagnostic table or the diagnostic chair so that it may capture an image of the subject at a fixed position although the ultrasound diagnosis apparatus 1000 moves. A detailed embodiment will be described with reference to FIG. 11.

The image analyzer 1200 acquires position information. The position information may include a spatial position of a probe with respect to a subject. The image analyzer 1200 may acquire the position information by recognizing a probe via the infrared imaging unit 1100. The position information may be represented by 3D coordinates.

For example, as the infrared imaging unit 1100 receives an infrared signal transmitted from an infrared LED, the image analyzer 1200 may analyze the received infrared signal and acquire the position information about the position of the probe. That is, when the infrared imaging unit 1100 senses the infrared signal transmitted from one or more infrared LED attached to the probe, the image analyzer 1200 may acquire the position information about the position of the probe by calculating a distance between the infrared imaging unit 1100 and the one or more infrared LED. Also, the image analyzer 1200 may measure and acquire the position information about the position of the probe in consideration of an angle at which the infrared signal is received and the intensity of the infrared signal.

In another embodiment, when the infrared imaging unit 1100 transmits an infrared signal and receives a signal reflected from a reflective sheet attached to a probe, the image analyzer 1200 may acquire the position information about the position of the probe by analyzing the received reflected signal. That is, the image analyzer 1200 may acquire the position information indicating a spatial position of the probe from a signal received by the infrared imaging unit 1100.

The image analyzer 1200 may acquire various pieces of information for an operation of the probe, in addition to the position information about the position of the probe. For example, the image analyzer 1200 may acquire information about a motion of the probe, which is changed depending on a user's handling, such as a linear direction of the movement of the probe, a scan direction of the probe, a rotation direction of the probe, or the like.

In the current embodiment, the infrared imaging unit 1100 may sense a change in an infrared signal or a reflected signal that is received from the infrared LED or the reflective sheet, depending on a motion of the probe. Thus, the image analyzer 1200 may analyze a linear direction of the movement of the probe, a scan direction of the probe, or a rotation direction of the probe, which is changed depending on a user's handling. The image analyzer 1200 may correctly determine a diagnostic part by using various pieces of information about a motion of the probe. An embodiment related to this will be described in detail with respect to FIG. 6.

In another embodiment, the image analyzer 1200 may use feature points of a subject sensed by the infrared imaging unit 1100 to acquire the position information about the position of the probe. For example, when the infrared imaging unit 1100 senses two positions of both shoulders of a patient (i.e., the subject) and two positions of the pelvis of the patient, i.e., four points, as the feature points, the image analyzer 1200 may recognize a frame structure of the subject, which is obtained by connecting the four points.

Furthermore, the image analyzer 1200 may divide the frame structure into a plurality of segments, and may match the position of the sensed probe to any one of the plurality of segments. The image analyzer 1200 may acquire a position of the matched segment as the information of the probe.

In another embodiment, the image analyzer 1200 may acquire position information about the position of a probe through a 3D labeling process. That is, the image analyzer 1200 may apply a 3D labeling algorithm to a subject image captured by the infrared imaging unit 1100. Thus, the image analyzer 1200 may distinguish a user's hand grasping the probe and the probe from the image of the subject to recognize them. Subsequently, the image analyzer 1200 may acquire the position information by recognizing the position of the sensed probe.

In another embodiment, the image analyzer 1200 may compare an image previously captured from an subject with anther image captured from both a probe and an subject to acquire position information about the position of the probe. That is, the image analyzer 1200 may store a first image captured from only the subject in advance before a user handles the probe, and may capture a second image when both the probe and the subject are sensed according to the handling of the user. Subsequently, the image analyzer 1200 may sense the probe by comparing the first image and the second image, and thus may acquire the position information about the position of the probe.

The image analyzer 1200 may acquire the position information about the position of the probe according to any one of various methods stated above, and may use the position information to determine a diagnostic part of the subject. The diagnostic part may be a portion of a subject that is a target object to which the probe transmits an ultrasonic signal. For example, the diagnostic part may be a portion of the subject, such as the brain, liver, heart, thyroid, womb, wrist, or the like.

In detail, by using the position information about the position of the probe with respect to the subject, the image analyzer 1200 may recognize a part of a target object to which an ultrasonic signal is transmitted through the probe and an echo signal is received therefrom. For example, the image analyzer 1200 may compare a frame structure of the subject with an average human body model, and may determine an organ of the average human body model as the diagnostic part based on the position information about the position of the probe.

The image analyzer 1200 may use probe information when determining the diagnostic part. The probe information may include various pieces of information about an outward shape or physical and mechanical characteristics of the probe, such as an arrangement of a transducer (a linear array, a convex array, a phased array, etc), the type of the probe (2D, 2.5D, 3D, etc), and the purpose of the probe (for heart diagnosis, for womb diagnosis, for thyroid diagnosis, etc.).

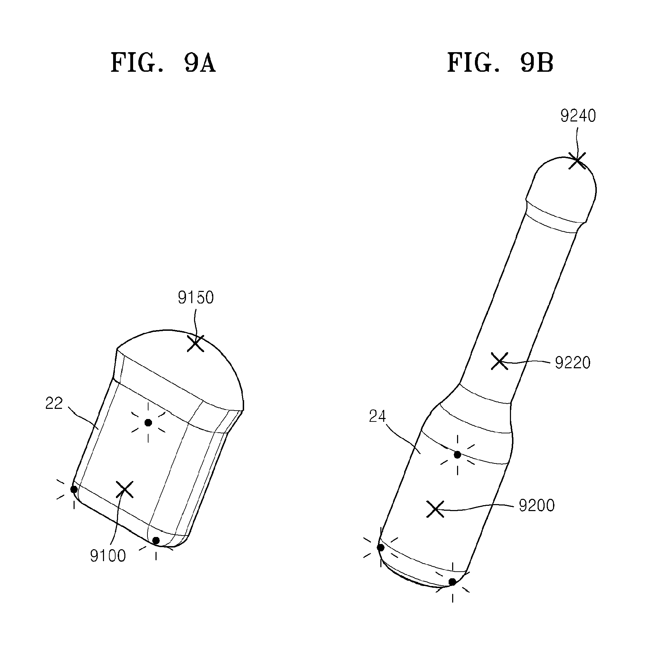

For example, although an ultrasonic signal is transmitted from the same position, the ultrasonic signal may be delivered to different diagnostic parts depending on the form of the probe, the length of the probe, etc. Thus, the image analyzer 1200 may consider both the probe information stored beforehand in the ultrasound diagnosis apparatus 1000 and the position information about the position of the probe. That is, the image analyzer 1200 may accurately determine the diagnostic part according to the probe information and the position information. An embodiment related to this will be described in detail with reference to FIG. 9.

As described above, the image analyzer 1200 may acquire the position information about the position of the probe, and may determine the diagnostic part by using the acquired position information.

The image processor 1300 generates various pieces of visual information which are output from the ultrasound diagnosis apparatus 1000 as graphic data. For example, the image processor 1300 may generate various pieces of information by using a graphic user interface (GUI), and may generate a body marker for the diagnostic part. The image processor 1300 may use 4D images, which are real time images, as well as 2D images and 3D images to generate the body marker.

In an embodiment, the image processor 1300 may use images stored beforehand according to various diagnostic parts to generate the body marker. Alternatively, the image processor 1300 may use images of the subject which are captured in real time to generate the body marker.

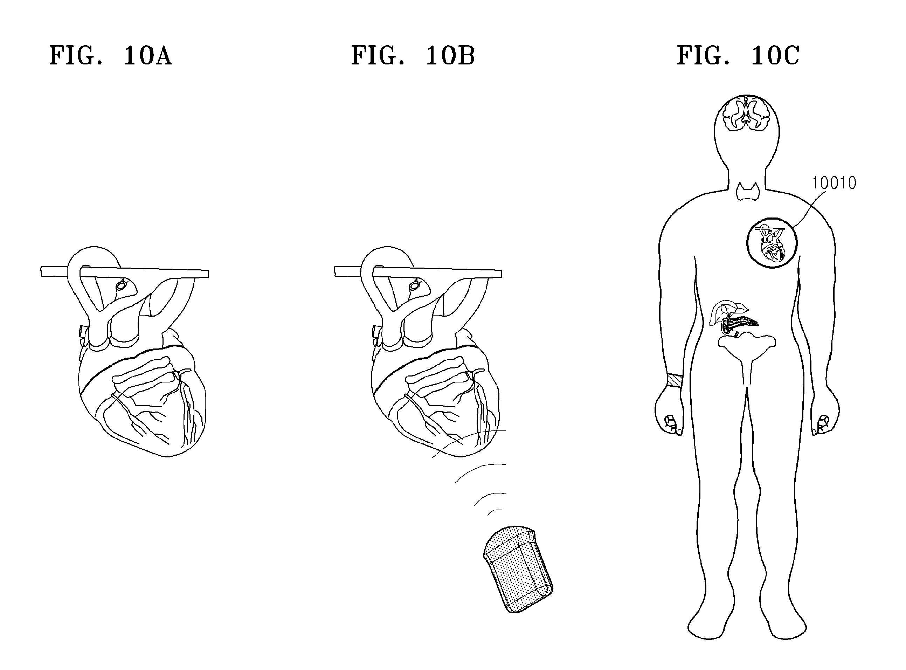

The body marker that is generated by the image processor 1300 may be an image for a diagnostic part, and may be also an image indicating both the diagnostic part and the probe. Also, the body marker may be an image including a visual effect with respect to a diagnostic part in an image of the subject. A detailed embodiment related to this will be described with reference to FIG. 10.

The display unit 1400 displays various pieces of graphic data and graphic information generated by the image processor 1300. For example, the display unit 1400 may output a body marker generated by the image processor 1300.

The display unit 1400 may include at least one selected from among a liquid crystal display (LCD), a thin film transistor LCD, an organic light-emitting diode (OLED), a flexible display, a 3D display, and an electrophoretic display. The ultrasound diagnosis apparatus 1000 may include two or more display units depending on an implementation type.

Moreover, when the display unit 1400 and a touch pad are implemented as a touch screen via a layer structure, the display unit 1400 may be used as an input unit in addition to an output unit. The display unit 1400 may detect a proximity touch as well as a real touch. The display unit 1400 implemented as a touch screen may sense a touch input (e.g., a touch and hold, a tap, a double tap, a flick, etc.) on an image output therefrom. Also, the display unit 1400 may sense a drag input from a point where a touch input is sensed and multiple touch inputs (e.g., a pinch input) on at least two points.

FIG. 3 is a block diagram illustrating an ultrasound diagnosis apparatus according to another embodiment of the present invention. The same descriptions as explained with reference to FIGS. 1 and 2 are not repeated.

An image processor 1300 includes a body marker generation module 1310 and an ultrasound image generation module 1330. The body marker generation module 1310 may generate a body marker as graphic data, as described with respect to the image processor 1300 of FIG. 2.

The ultrasound image generation module 1330 receives ultrasonic data generated from an echo signal received by a probe 20, and generates an ultrasound image by using the ultrasonic data. The ultrasound image generation module 1330 may generate an ultrasound image of the A mode, an ultrasound image of the B mode, and an ultrasound image of the M mode in a gray scale, and may also generate a Doppler image indicating a motion of a target object.

The display unit 1400 displays the body marker generated as described with reference to FIG. 2. Also, the display unit 1400 may display the ultrasound image generated by the ultrasound image generation module 1330. For example, the display unit 1400 may display an ultrasound image of a diagnostic part, which is a target object to or from which the probe 20 transmits or receives an ultrasonic signal, and may also display, together with the ultrasound image, a body marker generated with respect to the diagnostic part. The display unit 1400 may overlay the body marker on the ultrasound image to display them, and may display the ultrasound image and the body marker separately in different areas on a screen.

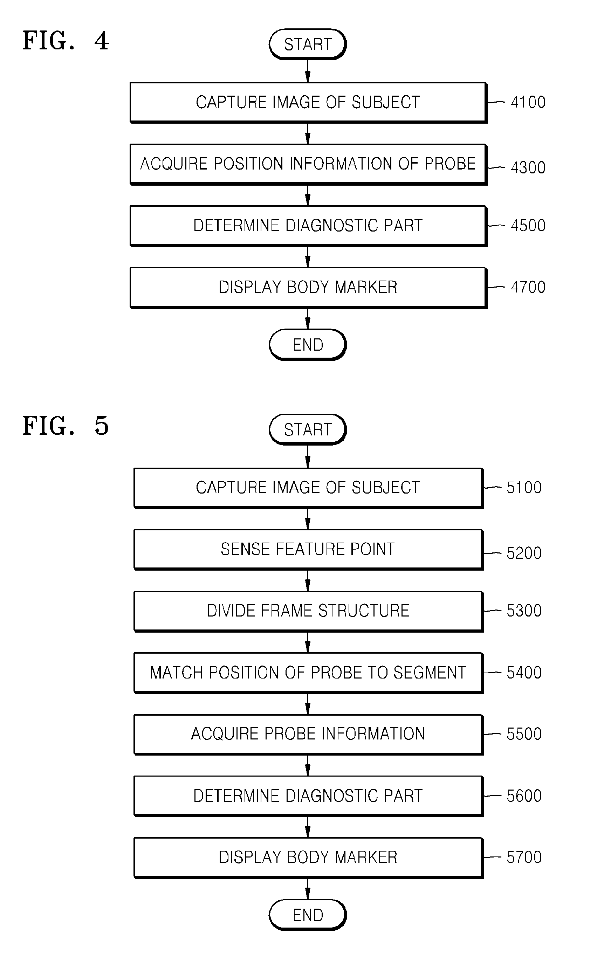

A method of generating a body marker by using the elements of the ultrasound diagnosis apparatus 1000 is described with reference to FIGS. 4 and 5.

FIG. 4 is a flowchart illustrating a method of generating a body marker, according to an embodiment of the present invention. The flowchart illustrate in FIG. 4 includes operations that are sequentially processed in the ultrasound diagnosis apparatus 1000, the infrared imaging unit 1100, the image analyzer 1200, the image processor 1300, and the display unit 1400, which are illustrated in FIG. 2 or FIG. 3. Thus, although omitted below, the above descriptions with respect to the elements illustrated in FIGS. 2 and 3 also apply to the flowcharts illustrated in FIGS. 4 and 5.

In operation 4100, the ultrasound diagnosis apparatus 1000 captures an image of a subject by using a 3D infrared sensor. The ultrasound diagnosis apparatus 1000 may transmit an ultrasonic signal to the subject and acquire information about a depth from the ultrasound diagnosis apparatus 1000 to the subject. Also, the ultrasound diagnosis apparatus 1000 may sense a feature point of the subject by using the 3D infrared sensor.

In operation 4300, the ultrasound diagnosis apparatus 1000 acquires position information about the position of a probe. The ultrasound diagnosis apparatus 1000 may acquire the position information about the position of the probe by using various methods as described with reference to FIG. 2, and may receive an ultrasonic signal from an ultrasonic LED or a reflection signal from a reflective sheet.

Also, the ultrasound diagnosis apparatus 1000 may acquire the position information through a 3D labeling process with respect to an image captured from the subject, and may compare two or more images captured from the subject to acquire the position information about the position of the probe.

Next, in operation 4300, the ultrasound diagnosis apparatus 1000 may acquire information about a motion of the probe, such as a linear direction of the movement of the probe, a scan direction of the probe, a rotation direction, or the like, in addition to the position information about the position of the probe.

In operation 4500, the ultrasound diagnosis apparatus 1000 determines a diagnostic part. The ultrasound diagnosis apparatus 1000 may use the position information indicating a spatial position of the probe with respect to the subject to determine the diagnostic part that is a target object to which a user transmits an ultrasonic signal.

In operation 4700, the ultrasound diagnosis apparatus 1000 displays a generated body marker. That is, the ultrasound diagnosis apparatus 1000 may generate a body marker according to the diagnostic part determined in operation 4500, and may display the generated body marker on the screen. The ultrasound diagnosis apparatus 1000 may display the body marker together with an ultrasound image.

FIG. 5 is a flowchart illustrating a method of generating a body marker, according to another embodiment of the present invention. The descriptions explained with reference to FIG. 4 are not repeated.

In operation 5100, the ultrasound diagnosis apparatus 1000 captures an image of a subject. In operation 5200, the ultrasound diagnosis apparatus 1000 senses a feature point of the subject. That is, the ultrasound diagnosis apparatus 1000 may sense one or more feature points in the subject's image captured by using an ultrasonic signal. The ultrasound diagnosis apparatus 1000 may sense two positions of both shoulders of the subject and two positions of the pelvis of the patent as feature points. However, the above-stated feature points are only examples for convenience of explanation, and the ultrasound diagnosis apparatus 1000 may sense various different positions of the subject as feature points.

In operation 5300, the ultrasound diagnosis apparatus 1000 acquires a frame structure of the subject and divides the acquired frame structure. First, the ultrasound diagnosis apparatus 1000 connects the feature points sensed in operation 5200 to form the frame structure. The ultrasound diagnosis apparatus 1000 may skeletonize the subject by generating the frame structure.

Also, the ultrasound diagnosis apparatus 1000 divides the frame structure into a plurality of segments. That is, the ultrasound diagnosis apparatus 1000 may divide the frame structure into a plurality of segments arranged in 2D. For example, the ultrasound diagnosis apparatus 1000 may divide a frame structure of the upper body of the subject into 36 segments forming a 6x6 matrix. The number of segments stated here is only an example and the present invention is not limited thereto.

In operation 5400, the ultrasound diagnosis apparatus 1000 matches a position of a probe to a segment. That is, the ultrasound diagnosis apparatus 1000 may acquire position information about the position of the probe as described in operation 4300 of FIG. 4, and may match the position information to any one of the plurality of segments obtained in operation 5300. Through the above-described matching process, the ultrasound diagnosis apparatus 1000 may accurately specify the position of the probe with respect to the subject.

In operation 5500, the ultrasound diagnosis apparatus 1000 acquires probe information. The probe information may include various pieces of information about a mechanical structure, such as the type of the probe, the form of the probe, and the like, as described above.

In operation 5600, the ultrasound diagnosis apparatus 1000 determines a diagnostic part by using the position of the probe and the probe information obtained in operation 5500. That is, the ultrasound diagnosis apparatus 1000 does not determine the diagnostic part in consideration of only the position of the probe, but may accurately specify, in consideration of the specification, form, or length of the probe, which part is a target object to and from which the probe transmits an ultrasonic signal and receives an echo signal.

In operation 5700, the ultrasound diagnosis apparatus 1000 displays a body marker. That is, the ultrasound diagnosis apparatus 1000 generates a body marker for the diagnostic part determined in operation 5600 and displays the generated body marker on a screen.

FIGS. 6A and 6B are diagrams illustrating processes of acquiring position information about the position of a probe 20, according to embodiments of the present invention.

FIG. 6A illustrates a process of acquiring the position information about the position of the probe 20 by using infrared LEDs 6100. An infrared imaging unit 1100 of the ultrasound diagnosis apparatus 1000 receives infrared signals 6110 that are transmitted from the infrared LEDs 6100 attached on the probe 20. The ultrasound diagnosis apparatus 1000 may acquire position information about the position of the probe 20 by analyzing the received infrared signals 6110. For example, the ultrasound diagnosis apparatus 1000 may acquire space coordinates of a central position of three infrared LEDs 6100 as the position information about the position of the probe 20.

The ultrasound diagnosis apparatus 1000 may acquire information about a motion of the probe 20, such as a direction of the movement of the probe 20, a scan direction of the probe 20, a direction of the rotation of the probe 20, or the like, in addition to the position information about the position of the probe 20. That is, the ultrasound diagnosis apparatus 1000 may sense a position and direction of movement of the probe 20 according to a change in positions of the three infrared LEDs 6100 attached on the probe 20.

In the embodiment illustrated in FIG. 6A, a user moves the probe 20 in the direction of an arrow 6150 to scan a subject. Thus, the ultrasound diagnosis apparatus 1000 may determine the direction of the movement of the probe 20 and the scan direction of the probe 20 by sensing the change in the positions of the three infrared LEDs 6100.

FIG. 6B illustrates a process of acquiring the position information about the position of the probe 20 by using a reflective sheet 6200. The infrared imaging unit 1100 of the ultrasound diagnosis apparatus 1000 transmits an infrared signal 6210 to the reflective sheet 6200, and receives a reflection signal 6220 reflected from the reflective sheet 6200 attached on the probe 20. The ultrasound diagnosis apparatus 1000 may acquire the position information about the position of the probe 20 by analyzing the reflection signal 6220.

As described in FIG. 6A, the ultrasound diagnosis apparatus 1000 may acquire information about a motion of the probe 20 in addition to the position information. A pattern for generating the reflection signal 6220 may be formed in the reflective sheet in advance. Thus, the ultrasound diagnosis apparatus 1000 may determine the motion of the probe 20 by sensing the movement and rotation of the pattern formed in the reflective sheet 6200. In the embodiment illustrated in FIG. 6B, the ultrasound diagnosis apparatus 1000 may determine the motion of the probe 20 which rotates in an arrow direction 6250 by sensing a rotation of the reflective sheet 6200.

FIG. 7 is a diagram illustrating a process of acquiring position information about the position of a probe 20, according to another embodiment of the present invention.

The ultrasound diagnosis apparatus 1000 may capture an image of an subject 7050, and may sense feature points 7100 of the subject 7050. In the embodiment illustrated in FIG. 7, the ultrasound diagnosis apparatus 1000 may sense two positions of both shoulders of the subject 7050 and two positions of the pelvis of the subject as the feature points 7100. However, the positions are only examples, and the present invention is not limited thereto.

The ultrasound diagnosis apparatus 1000 connects the feature points 7100 to acquire a frame structure 7200. In the embodiment illustrated in FIG. 7, the ultrasound diagnosis apparatus 1000 may use a tetragon obtained by connecting the feature points 7100 as the frame structure 7200 of the subject.

Next, the ultrasound diagnosis apparatus 1000 divides the skeletonized frame structure 7200 into a plurality of segments. In the embodiment illustrated in FIG. 7, the ultrasound diagnosis apparatus 1000 divides the frame structure 7200 into tetragonal segments forming a 6x6 array. However, the present invention is not limited thereto. For example, the ultrasound diagnosis apparatus 1000 may divide the frame structure 7200 into a much larger or smaller number of segments, and may divide the frame structure 7200 into a continuous array of triangular segments and inverted triangular segments.

The ultrasound diagnosis apparatus 1000 may select any one of the plurality of segments as the position information about the position of the probe 20 by using the position of the sensed probe 20. In the embodiment illustrated in FIG. 7, the ultrasound diagnosis apparatus 1000 may match the center of a segment 7250 with the position information about the position of the probe 20. Thus, the ultrasound diagnosis apparatus 1000 may reduce an error of the position information about the position of the probe 20, and may measure an accurate position of the probe 20 with respect to the subject.

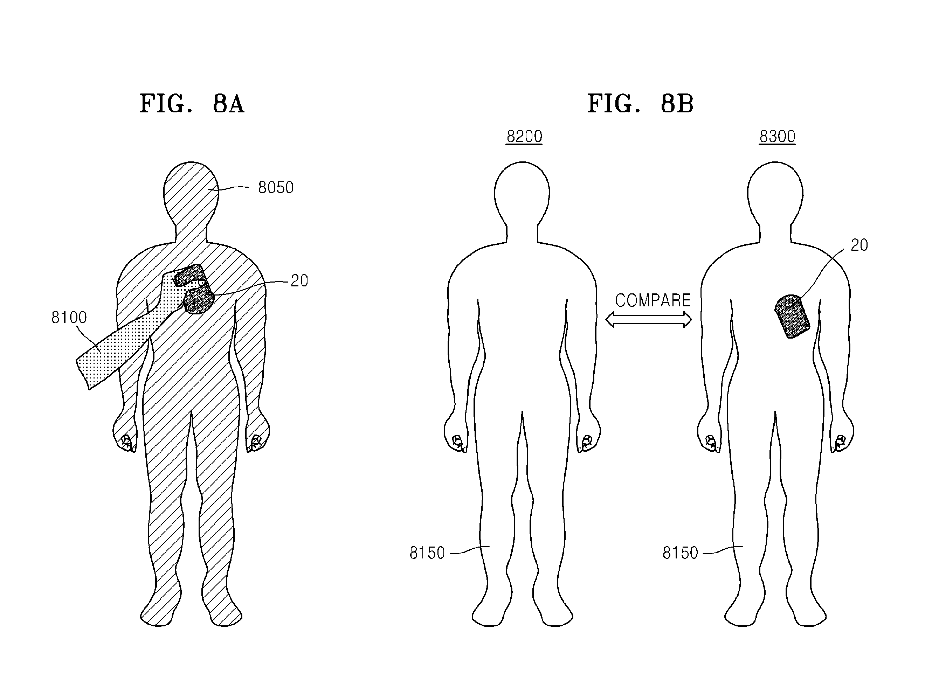

FIGS. 8A and 8B are diagrams illustrating processes of acquiring position information about the position of a probe 20, according to other embodiments of the present invention.

As described above, a 3D modeling process or an image comparison process corresponds to another embodiment in which the ultrasound diagnosis apparatus 1000 acquires position information about the position of a probe.

Referring to FIG. 8A, the ultrasound diagnosis apparatus 1000 captures an image of a user's hand 8100 holding the probe 20 and an image of an subject 8050. Next, the ultrasound diagnosis apparatus 1000 applies a 3D labeling algorithm to a captured image. As a result, the ultrasound diagnosis apparatus 1000 may distinguish the subject 8050, the user's hand 8100, and the probe 20 from each other to recognize them. Next, the ultrasound diagnosis apparatus 1000 may track the recognized probe 20, and may change a diagnostic part according to a path through which the probe 20 moves.

Referring to FIG. 8B, the ultrasound diagnosis apparatus 1000 generates a first image 8200 captured from a subject 8150 in advance before a user diagnoses the subject 8150 by using the probe 20. Next, the ultrasound diagnosis apparatus 1000 may generate a second image 8300 captured from both the probe 20 and the subject 8150, and may compare the first image 8200 and the second image 8300 to each other.

The ultrasound diagnosis apparatus 1000 may identify and recognize the probe 20 through the above-stated image comparison process. Like in FIG. 8A, the ultrasound diagnosis apparatus 1000 may determine a diagnostic part according to a change in position information of the recognized probe 20.

FIGS. 9A and 9B are diagrams illustrating processes of determining a diagnostic part, according to an embodiment of the present invention.

Referring to FIG. 9A, the ultrasound diagnosis apparatus 1000 senses a position 9100 that is a center of three infrared LEDs attached on a probe 22. Next, the ultrasound diagnosis apparatus 1000 may acquire probe information about the probe 22 selected by a user to diagnose a subject. For example, the ultrasound diagnosis apparatus 1000 may acquire information about the form and length of the activated probe 22.

Next, in consideration of the form and length of the probe 22, the ultrasound diagnosis apparatus 1000 may acquire as position information about the position of the probe 22 a position 9150 separated from the position 9100 by a certain distance in a predetermined direction,. That is, in consideration of the probe information as well as the position of the probe 22, the ultrasound diagnosis apparatus 1000 may acquire the position 9150 as the position information.

Referring to FIG. 9B, the ultrasound diagnosis apparatus 1000 senses a position 9200 that is a center of infrared LEDs attached on a probe 24. Next, the ultrasound diagnosis apparatus 1000 may acquire probe information about the probe 24. That is, the ultrasound diagnosis apparatus 1000 may acquire information about the form and length of the probe 24 having a length that is larger than that of the probe 22 of FIG. 9A.

Next, the ultrasound diagnosis apparatus 1000 may acquire a position 9240 as position information, the position 9240 being determined in consideration of the probe information from the position 9200. That is, as the probe 22 of FIG. 9A and the probe 24 of FIG. 9B have different forms and lengths, the ultrasound diagnosis apparatus 1000 may not acquire as the position information a position 9220 corresponding to the position 9150 of FIG. 9A but the position 9240.

According to the embodiments described above, the ultrasound diagnosis apparatus 1000 may acquire accurate position information in consideration of the mechanical characteristics, form, and type of a probe. In other words, the ultrasound diagnosis apparatus 1000 may acquire different positions as position information based on the type of the probe although the probe is sensed at the same position.

FIGS. 10A through 10C are diagrams illustrating processes of displaying a body marker, according to an embodiment of the present invention. In FIGS. 10A, 10B, and 10C, the ultrasound diagnosis apparatus 1000 determines a heart of a subject as a diagnostic part.

Referring to FIG. 10A, the ultrasound diagnosis apparatus 1000 displays an image of the heart, which is the diagnostic part, as a body marker. That is, the ultrasound diagnosis apparatus 1000 may inform a user that a target object is the heart by displaying an exemplary image of the heart.

Referring to FIG. 10B, the ultrasound diagnosis apparatus 1000 may display an image of the probe in addition to the image of the heart as the body marker. The ultrasound diagnosis apparatus 1000 may provide position information about the position of the probe in addition to a diagnostic part determined according to the position information as the body maker. Although in the embodiment illustrated in FIGS. 10A through 10C, a convex array-type probe diagnoses the heart under the right side of the heart, the ultrasound diagnosis apparatus 1000 may display a different form of body marker according to the type and position of a probe.

Referring to FIG. 10C, the ultrasound diagnosis apparatus 1000 may display the body marker by visually distinguishing the heart, which is the diagnostic part, from other parts in an entire exemplary image of the subject. That is, as illustrated in FIG. 10C, from among various parts of the subject, such as the brain, thyroid, heart, liver, womb, wrist, or the like, the ultrasound diagnosis apparatus 1000 determines the heart as the diagnostic part based on the position information about the position of the probe. Next, the ultrasound diagnosis apparatus 1000 may provide to a user as the body marker graphic data in which a circular marker 10010 is displayed on the heart (i.e., the diagnostic part) in the whole image of the subject.

Besides the embodiment illustrated in FIGS. 10A through 10C, the ultrasound diagnosis apparatus 1000 may allow a user to immediately recognize a diagnostic part by providing various types of body markers to the user.

FIG. 11 is a diagram illustrating an arrangement of a 3D infrared sensor, according to an embodiment of the present invention.

As shown in FIG. 11, an infrared imaging unit 1100 is attached to a diagnostic table 11000. Thus, although a movable ultrasound diagnosis apparatus 1000 moves according to the handling of a user, the infrared imaging unit 1100 captures an image of an subject 11050 at a certain position.

That is, the ultrasound diagnosis apparatus 1000 may capture an image of the subject 11050 and an image of a probe 20 while being attached to a structure on which the subject 11050 is positioned, such as the diagnostic table 11000 or a diagnostic chair.

The embodiments of the present invention may be written as computer programs and may be implemented in general-use digital computers that execute the programs using a computer readable recording medium. In addition, a structure of data used in the above-described method may be recorded in a computer readable recording medium through various methods. Examples of the computer readable recording medium include magnetic storage media (e.g., ROMs, RAMs, universal serial buses (USBs), floppy disks, hard disks, etc.), optical recording media (e.g., CD-ROMs, or DVDs), and storage media such as PC interfaces (e.g., PCI, PCI-express, WiFi, etc.).

As described above, the body marker generation method and the ultrasound diagnosis apparatus using the same may effectively generate and provide a body marker indicating an anatomical position of a diagnostic part during ultrasound diagnosis. That is, a user may immediately and intuitively recognize information about a diagnostic part that is changed by handling and moving a probe.

Thus, the accuracy of an ultrasound diagnosis may be improved, and the user may not unnecessarily handle a control panel of the ultrasound diagnosis apparatus to change the diagnostic part.

It should be understood that the exemplary embodiments described herein should be considered in a descriptive sense only and not for purposes of limitation. Descriptions of features or aspects within each embodiment should typically be considered as being available for other similar features or aspects in other embodiments. While one or more embodiments of the present invention have been described with reference to the figures, it will be understood by those of ordinary skill in the art that various changes in form and details may be made therein without departing from the spirit and scope of the present invention as defined by the following claims.

## Claims

1. An ultrasound diagnosis apparatus for generating a body marker, the ultrasound diagnosis apparatus comprising;
an infrared imaging unit for capturing an image of a subject by using a three dimensional (3D) infrared sensor;
an image analyzer for acquiring position information indicating a position of a probe with respect to the subject and for determining a diagnostic part of the subject based on the position information, the diagnostic part being a target object to which the probe transmits an ultrasonic signal;
an image processor for generating a body marker for the diagnostic part; and
a display unit for displaying the body marker.

2. The ultrasound diagnosis apparatus of claim 1, wherein the image analyzer measures at least one of a linear movement direction of the probe, a scan direction of the probe, and a rotation direction of the probe.

3. The ultrasound diagnosis apparatus of claim 1, wherein the infrared imaging unit receives a plurality of infrared signals through the 3D infrared sensor, the infrared signals being emitted from a plurality of infrared light emitting diodes (LEDs) attached on the probe, and the image analyzer measures the position information from the plurality of infrared signals.

4. The ultrasound diagnosis apparatus of claim 1, wherein the infrared imaging unit transmits an infrared signal by using the 3D infrared sensor and receives a reflection signal generated when the infrared signal is reflected by an infrared reflective sheet attached on the probe, and the image analyzer measures the position information from the reflection signal.

5. The ultrasound diagnosis apparatus of claim 4, wherein the infrared reflective sheet comprises a pattern for reflecting the infrared signal to generate the reflection signal.

6. The ultrasound diagnosis apparatus of claim 1, wherein the infrared imaging unit senses a plurality of feature points of the subject, and the image analyzer connects the plurality of feature points to each other to acquire a frame structure of the subject.

7. The ultrasound diagnosis apparatus of claim 6, wherein the image analyzer divides the frame structure into a plurality of segments, and matches the position of the probe to any one of the plurality of segments.

8. The ultrasound diagnosis apparatus of claim 1, wherein the image analyzer acquires the position information through a 3D labeling process for the probe.

9. The ultrasound diagnosis apparatus of claim 1, wherein the image analyzer compares a first image captured from the subject in advance and a second image captured from both the probe and the subject to acquire the position information.

10. The ultrasound diagnosis apparatus of claim 1, wherein the image analyzer acquires probe information indicating at least one of an arrangement of a transducer included in the probe, the type of the probe, and the purpose of the probe, and determines the diagnostic part based on the position information and the probe information.

11. The ultrasound diagnosis apparatus of claim 1, wherein the display unit displays an image indicating the diagnostic part and an image indicating the probe as the body marker.

12. The ultrasound diagnosis apparatus of claim 1, wherein the display unit displays an image in which the diagnostic part is distinguished visually from other parts in an exemplary image of the subject, as the body marker.

13. The ultrasound diagnosis apparatus of claim 1, wherein the 3D infrared sensor is attached to a diagnostic table or a diagnostic chair on which the subject is positioned.

14. A body marker generation method used by an ultrasound diagnosis apparatus, the body marker generation method comprising:
capturing an image of a subject by using a three dimensional (3D) infrared sensor;
acquiring position information indicating a position of a probe with respect to the subject;
determining a diagnostic part of the subject based on the position information, the diagnostic part being a target object to which the probe transmits an ultrasonic signal; and
displaying a body marker for the diagnostic part.

15. A non-transitory computer-readable recording medium having recorded thereon a program for executing the method of claim 14.
